# EUROPEAN PATENT APPLICATION

(11) **EP 1 285 660 A1**
(43) Date of publication of application: **26.02.2003**
(21) Application number: 01932310.4
(22) Date of filing: 28.05.2001
(51) Int. Cl.: A61K 33/30, A61K 47/16, A61K 9/20, A61K 9/48, A61K 9/14, A61K 9/16, A61P 3/12

(54) **ZINC-SUPPLEMENTARY COMPOSITIONS FOR ORAL ADMINISTRATION**

(30) Priority: 31.05.2000 JP 2000161444
(71) Applicant: Hamari Chemicals Co., Ltd., Osaka-shi, Osaka 533 (JP)
(72) Inventor: NISHIMURA, Yasuhiro, Takarazuka-shi, Hyogo 665-0003 (JP); MATSUKURA, Takefumi, Osaka Sayama-shi, Osaka 589-0005 (JP)
(74) Representative: Kinzebach, Werner, Dr.
(86) International application number: JP0104478
(87) International publication number: WO01091762

(57) **Abstract**

A composition for providing zinc through oral administration comprises a mixture of a physiologically acceptable zinc salt and a dipeptide selected from the group consisting of L-carnosine, L-anserine, L-valenine and L-homocarnosine at a dipeptide to zinc molar ratio of at least 0.5, or a complex of zinc and the peptide, or a mixture of the dipeptide and the complex at a dipeptide to complex molar ratio of at least 0.5.

## Description

### BACKGROUND OF THE INVENTION

This invention relates to a composition for enhancing the absorption of zinc through the intestinal tract upon oral administration or ingestion which finds use as a medicament in the treatment of zinc deficiency or as a dietary supplement for providing zinc.

Zinc is an essential trace element for a living body and plays an important role to keep the body alive. A comprehensive discussion on zinc including its occurrence forms and functions in the living body, symptoms associated with zinc deficiency, the absorption mechanism of zinc through the intestinal tract and absorption inhibitors thereof may be found in C.T. Walsh et al., Enviomental Health Perspective 102 (1994): Supplement 2, 5-56. It is known that more than 300 zinc-containing enzymes are present in the living body and many such enzymes contain zinc in their active center. Representative examples of the enzymes include alkali phosphatase, carbonic anhydrase, carboxy peptitase, and alcohol dehydrogenase. Zinc is also contained in a protein that activates DNA and RNA replication. Here zinc forms a unique domain called zinc finger so as to express the replication function by the protein. Although only about 3g of zinc is contained in a living human body, its deficiency affects various body functions sytemically and may develop dysgeusia, dysosmia, growth supression, hypogonadism, immunodeficiency, mental and nerval disorders.

In recent years, low calorie diets are becoming popular among younger generations. These people are taking a small quantity a specific food such as instant foods or fast foods and live on unbalanced diets. Wheat flour for noodles contains phytic acid which remarkably inhibits absorption of zinc contained in foods through the intestinal tract. Among various food additives, polyphosphates and carboxymethylcellulose are known as a zinc absorption inhibitor. Many of dysgeusia patients due to zinc deficiency are said to be on such unbalanced diets. Thus, there are a number of factors in these days which can lead to zinc deficiency. Some medicines of frequent use strongly bind zinc as a chelate to inhibit its absorption. Zinc deficiency can be caused by a long term administration of such medicines.

Zinc deficiency may be prevented by taking well balanced meals including zinc-rich foods. In addition, it is necessary to provide some means for promoting absorption of zinc through the intestinal tract.

Zinc is absorbed through the intestinal tract into the body. In this process, zinc in the form of a chelate with a low molecular weight carrier substance is transported from the intestinal lumen through the brush border membrane into intestinal cells, temporarily stored there in the form of zinc thioneine, and then released into the blood stream when necessary to deliver zinc to the living tissue. It has not been well elucidated yet what molecules are carriers for zinc absorption. Amino acids, di- and tripeptides have been postulated to be such carrier because a high protein diet enhances the absorption of zinc. Cysteine-rich intestinal protein (CRIP) found in intestinal walls and 2-picolinic acid found in pancreatic juice and bile are also said to be a carrier of zinc transportation.

There are a number of reports to date on carrier substances which form a chelate with zinc to promote the absorption of zinc through the intestinal tract. Examples of such substances include L-glutamic acid (JP-A-57082318), naturally occurring amino acids, and di-, tri- or tetrapeptides thereof (JP-A-63502749), sugar phosphate esters (JP-A-02249468), and hydropyrones (JP-A-60115564). Picolinic acid and prostaglandins have also been reported to be effective as a carrier although not sufficiently proven.

The zinc carrier substances must be not only highly effective but also highly reliable in safety. The carrier substances which have been reported to date are not satisfactory in both effectiveness and safety. A need exists for improvement in the effectiveness and safety of the carrier substance.

### SUMMARY OF THE INVENTION

The present invention has its basis on a finding that the above need may be met by selecting L-carnosine, L-homocarnosine, L-anserine, L-valenine, their acid addition salts or esters as a carrier substance for zinc.

Based on this finding, the present invention provides a composition for orally providing zinc to a human subject comprising a mixture of a physiologically acceptable zinc salt and a dipeptide selected from the group consisting of L-carnosine, L-homocarnosine, L-anserine, L-valenine, an acid addition salt thereof and an ester thereof at a dipeptide to zinc molar ratio of at least 0.5, or a complex of zinc and said dipeptide.

The composition of the present invention may be administered in the form of tablets, capsules, powders, granules or other solid dosage forms for oral administration as a medicament for treating zinc deficiency or as a dietary supplement for providing zinc.

### BRIEF DESCRIPTION OF THE DRAWINGS

Fig. 1 is a graph showing the bioavalability of zinc at varying L-carnosine to zinc molar ratios, and
Fig. 2 is a graph showing dose-dependent absorption of zinc by the oral administration of a mixture of L-carnosine and zinc acetate.

### DETAILED DISCUSSION

L-carnosine, otherewise called N-β-alanyl-L-histidine is a naturally occurring dipeptide found in muscles of human and other vertebrates. L-anserine, otherwise called N-β-alanyl-3-methyl-L-histidine and L-valenine, otherwise called N-β-alanyl-1-methyl-L-histidine are also a naturally occurring dipeptide found in muscles of vertebrates other than humans. These dipeptides are present, for instance, in meat soups and do not have known toxicity to the living body. L-homocanosine is a dipeptide between L-histidine and γ-aminobutyric acid (GABA). Amino acids and peptides which have been hitherto known as a carrier substance for enhancing absorption of zine from the intestinal tract are all α-amino acids and peptides thereof. Use of dipeptide containing a β- or γ-amino acid has not been known.

L-carnosine, L-homocarnosine, L-anserine and L-valenine may be obtained by extracting from muscle tissues or by the chemical synthesis from the amino acids constituting the dipeptide. An acid addition salt of the dipeptide such as hydrochloride or an ester such as methyl, ethyl or other lower alkyl esters may also be employed.

The zinc salt to be co-administered with the dipeptide must be capable of ionizing into zinc ions in the digestive tract to form a chelate or complex with the dipeptide. The zinc salt in general, therefore, must be easily soluble in water or in digestive juice and also physiologically tolerable in a long term administration. Among a number of known water-soluble zinc salts, sulfate, acetate and lactate are preferred. Zinc-rich extracts such as, for example, meat extracts from cattle, swine, sheep or poultry, extracts from internal organs (liver, pancreas and prostate) of these animals, extracts from oysters or crabs, or extracts from seaweed *(Laminavia, Undaria, Hizikia and Porphyra)* can be a supply of zinc salts.

The molar ratio of dipeptide to zinc atom must be at least 0.5 (0.5:1). The bioavailability of zinc was found to be increased as the ratio of dipeptide to zinc atom increases. Since excessive ratios of dipeptide to zinc atom are not preferable from economical point of view, a ratio from 1 to 30, particularly 1 to 10 is suitable.

The dipeptides as mentioned above are known to form a complex with zinc. See, e.g. JP-B-03005387. The complex may be used alone or in combination with the dipeptide at a ratio of dipeptide to zinc complex of at least 0.5.

The composition of the present invention may be processed into solid dosage forms suitable for oral administration such as tablets, capsules, powders or granules using the conventional method. Enteric coated preparations are preferable to avoid influence of acidic gastric juice. Daily requirement of zinc uptake is reported to be 10 to 15 mg for children, 15 mg, ideally 20 to 100 mg for adult men, 15 mg for adult women, 20 mg for pregnant women, and 25 mg for lactational women, respectively. The dose of the above solid dosage forms may easily be determined based on the above daily requirement of zinc. Since the safety of zinc has been well ascertained, there is little concern about the toxicity of excessive administration of the composition of the present invention.

The following experiments will demonstrate the effect of the dipeptides on the enhancement of adsorption of zinc through the digestive tract according to the present invention.

### EXPERIMENT:

### 1. Increased bioavailability of zinc by co-administration of dipeptide or administraion of complex of zinc and dipeptide

Wistar rats of 8 weeks age were used in this experiment. The rats were fasted for 24 hours before administration of zinc acetate alone, a mixture of zinc acetate and a dipeptide listed in Table 1 at a molar ratio of dipeptide to zinc of 1:1, or a complex of zinc and L- or D-carnosine at a molar ratio of dipeptide to complex of 1:1. Each test composition was administered to the duodenum of ether-anesthetized rat as a solution or suspension in physiological saline. The dose of the composition was 10 mg/kg body weight as zinc in each case. Blood samples were collected before administration, and 1,3,5 and 7 hours after the administration. Plasma levels of zinc were determined using Zn Test Wako Kit (available from Wako Pure Chemical Industries, Ltd.). The absorbed amount of zinc was determined from the area under the curve (AUC) of blood level of zinc vs. time curve up to 7 hours and compared with the AUC obtained from intravenous administration of zinc acetate at a dose of 1 1 mg/kg body weight. The bioavailability (BA) of zinc was calculated by the above comparison. The results are shown in Table 1 below.

**Table 1**

| Sample | N | BA (%) |
|---|---|---|
| Zinc acetate | 71 | 8.6 ± 0.3 |
| Zinc acetate+L-carnosine | 5 | 13.6 ± 2.1^{***} |
| Zinc acetate+L-anserine | 5 | 12.2 ± 1.4^{***} |
| Zinc acetate+L-carnosine | 5 | 11.6 ± 0.8^{**} |
| methyl ester | | |
| Zinc acetate+L-homocarnosine | 5 | 12.5 ± 1.2^{**} |
| L-carnosine zinc complex | 5 | 11.7 ± 0.4^{**} |
| D-carnosine zinc complex | 5 | 6.7 ± 0.8 |

| | | |
|---|---|---|
| Test of significance relative to zinc acetate: ***p < 0.001 | | |
| ** p < 0.01 | | |

As shown in Table 1, co-administration of L-carnosine, L-anserine, L-carnosine methyl ester or L-homocarnosine increased the bioavailability of zinc compared with the administration of zinc acetate alone. When the dipeptide was administered as the complex with zinc instead of mixture, L-carnosine exhibited a similar absorption promoting action but not with D-carnosine.

### 2. Effect of the molar ratio of L-carnosine to zinc on the bioavailability

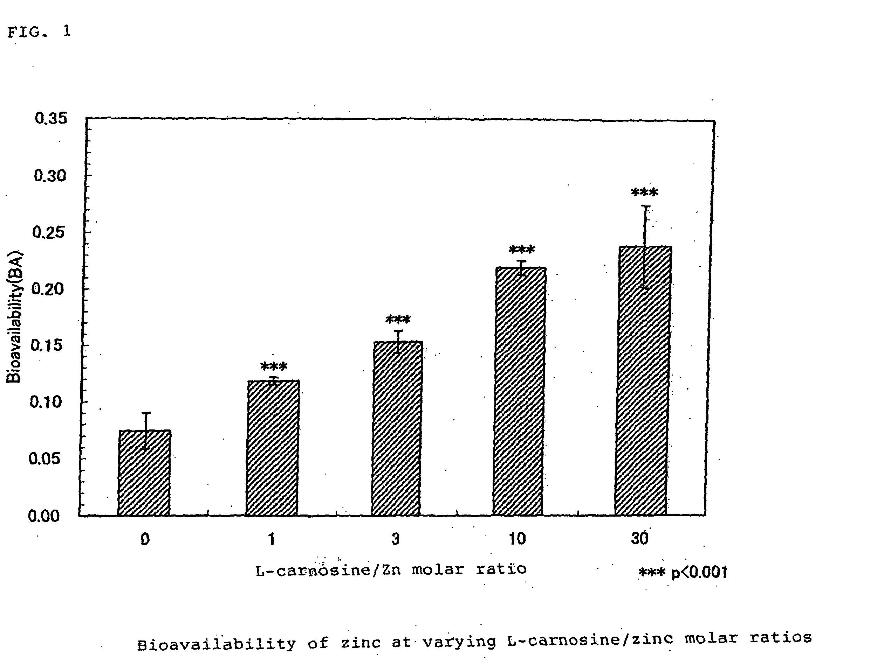

Using the same method as above, the bioavailability of zinc was determined at varying molar ratios of L-carnosine to zinc from zero to 30. The results are shown in Fig. 1 of the accompanying drawings. As shown, the bioavailability increased with increase in the molar ratio.

### 3. Dose dependency of absorption of zinc

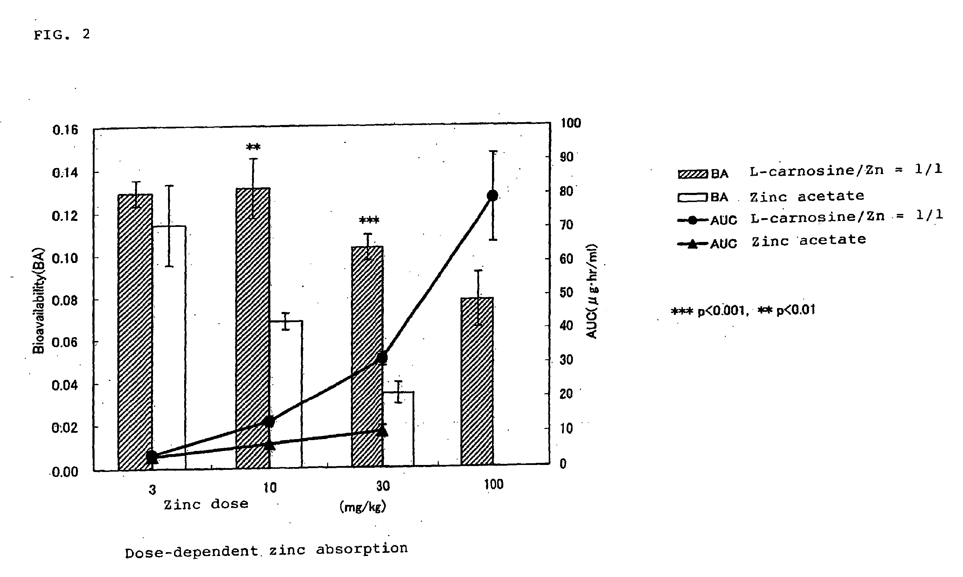

The dose of a 1:1 molar mixture of L-carnosine and zinc acetate was varied from 3 mg/kg to 100 mg/kg in terms of zinc. Analogous to Part 1, AUCs and bioavalabilities were determined and compared with those of administration of zinc acetate alone. The results are shown in Fig. 2 of the accompanying drawing. As shown, the total amount of absorbed zinc represented by the AUC clearly increased in dose dependent manner by the co-administration of L-carnosine compared with the administration of zinc acetate alone, while the absorption rate represented by the bioavailability reached peak at a dose of 10 mg/kg and then decreased less sharply than the administration of zinc acetate alone.

### EXAMPLES:

The following examples are given to illustrate the present invention without limiting thereto.

### Example 1

A powder preparation was prepared by the conventional method from the materials shown in the following formulation.

| Formulation: | |
|---|---|
| L-Carnosine | 50 mg |
| Zinc acetate | 40 mg |
| Lactose | 900 mg |
| Hydroxypropylcellulose | 5 mg |
| Anhydrous silica | 5 mg |
| Total | 1000 mg |

### Example 2

A granule preparation was produced by the conventional method from the materials shown in the following formulation.

| Formulation: | |
|---|---|
| L-Carnosine zinc complex (1:1) | 200 mg |
| Lactose | 540 mg |
| Corn starch | 240 mg |
| Hydroxypropylcellulose | 10 mg |
| Total | 1000 mg |

### Example 3

The materials shown in the following formulation were processed into granules by the conventional method and then compressed into tablets.

| Formulation: | |
|---|---|
| L-Carnosine zinc complex (1:1) | 100.0 mg |
| Lactose | 50.0 mg |
| Corn starch | 23.5 mg |
| Carboxymethylcellulose calcium | 4.0 mg |
| Methylcellulose | 2.0 mg |
| Magnesium stearate | 0.5 mg |
| Total | 180 mg |

### Example 4

The materials shown in the following formulation were processed into granules by the conventional method and then filled in #2 gelatin capsule to obtain a capsule preparation.

| Formulation: | |
|---|---|
| L-Anserine | 50 mg |
| Zinc sulfate | 120 mg |
| Lactose | 570 mg |
| Corn starch | 250 mg |
| Hydroxypropylcellulose | 10 mg |
| Total | 1000 mg |

## Claims

1. A composition for providing zinc through oral administration comprising a mixture of a physiologically acceptable zinc salt and a dipeptide selected from the group consisting of L-carnosine, L-anserine, L-homocarnosine and L-valenine, an acid addition salt and an ester of said dipeptide at a dipeptide to zinc molar ratio of at least 0.5; a complex of zinc and said dipeptide; or a mixture of said zinc complex and said dipeptide at a dipeptide to zinc complex molar ratio of at least 0.5.

2. A composition according to claim 1 wherein said dipeptide is L-carnosine and said zinc salt is selected from zinc sulfate, zinc acetate or zinc lactate.

3. A composition according to claim 1 wherein said complex is L-carnosine zinc complex.

4. A composition according to any of claims 1-3 in the form of tablets, capsules, powders, granules or other solid dosage forms.

5. A composition according to claim 4 which is a medicament for treating a disease associated with zinc deficiency.

6. A composition according to claim 1 which is a dietary supplement for providing zinc as a trace nutrient.
